# EUROPÄISCHE PATENTSCHRIFT

(11) **EP 2 995 339 B1**
(45) Veröffentlichungstag und Bekanntmachung des Hinweises auf die Patenterteilung: **08.01.2020**
(21) Anmeldenummer: 15173650.1
(22) Anmeldetag: 24.06.2015
(51) Int. Cl.: A61M 25/10

(54) **BALLONKATHETER**
BALLOON CATHETER
CATHETER A BALLONNET

(30) Priorität: 15.09.2014 US 201462050212 P; 15.09.2014 US 201462050211 P
(43) Veröffentlichungstag der Anmeldung: 16.03.2016
(73) Patentinhaber: Biotronik AG, 8180 Bülach (CH)
(72) Erfinder: Moehl, Raimund, 8127 Forch (CH); Filippi, Michael, 8037 Zürich (CH); Hofmann, Andreas, 91322 Gräfenberg (DE); Quint, Bodo, 79802 Dettighofen (DE)
(74) Vertreter: Randoll, Sören

(56) Entgegenhaltungen:
- EP-A1- 2 450 010
- WO-A2-2014/179767
- US-A1- 2009 018 501
- US-A1- 2013 237 950

## Beschreibung

Die Erfindung betrifft einen Ballonkatheter mit einem medikamentenbeschichteten Ballon, der in einem Ausgangszustand mit einer verschieblichen Schutzhülle bedeckt ist, auf einem langgestreckten Katheterkörper, der ein distales und ein proximales Ende hat. Sie betrifft des Weiteren einen Ballon zur Herstellung eines solchen Ballonkatheters.

Die Ballone sind ein entscheidendes Funktionselement von Ballonkathetern, wie sie (u. a.) zur Dilatation von verengten Gefäßen und zum Einbringen und Expandieren von Stents in Gefäßen oder Hohlorganen benutzt werden. Ihre zuverlässige Funktion ist von höchster Bedeutung für das Gelingen entsprechender Eingriffe.

Seit einiger Zeit versucht man, die Nachhaltigkeit der mit Ballonkathetern ausgeführten Dilatation verengter Gefäße zu vergrößern und insgesamt deren Performance zu verbessern, indem man auf den Ballon eine geeignete Medikamentenbeschichtung aufbringt. Ballone mit einer derartigen Beschichtung, die auch als DEB (Drug-Eluting Balloons) bezeichnet werden, sind Stand der Technik und im klinischen Einsatz; vgl. etwa http://www.biotronik.de/en/uk/24250.

Medikamentenbeschichtete Ballone haben den Nachteil, dass sie auf dem Weg zum Behandlungsort teilweise Medikament verlieren können. Das heisst, dass der Wirkstoffanteil am Behandlungsort variiert und dass die gelösten Partikel an anderen Orten undefiniert abgelagert werden können. Die Wirkung der Medikamentenbeschichtung ist also in gewissem Grade schwer kontrollierbar.

US 2009/0018501 A1 beschreibt dahingehend einen Ballonkatheter mit einem Außenschaft.

Der Erfindung liegt die Aufgabe zugrunde, einen Ballonkatheter der oben genannten Art bereitzustellen, der weitgehend ohne Nebenwirkungen eingesetzt werden kann, die aus abgelösten Teilen der Medikamentenbeschichtung resultieren.

Diese Aufgabe wird durch einen Ballonkatheter mit den Merkmalen des Anspruchs 1 gelöst. Zweckmäßige Fortbildungen des Erfindungsgedankens sind Gegenstand der abhängigen Ansprüche.

Die Erfindung schließt im Wesentlichen die folgenden Überlegungen/Aspekte ein:
Die Beschichtung des Ballons soll bis zum Behandlungsort mit einem entfernbaren Schutz versehen werden. Der Blutfluss soll solange unterbrochen werden, bis die Partikel, die sich möglicherweise bei der Balloninflation gebildet haben, durch den Ballondruck in die Gefäßwand eingebettet sind.

In einer konstruktiven Ausführung hat der Ballonkatheter einen einzelnen Ballon, der einen unbeschichteten distalen Abschnitt und einen beschichteten Abschnitt aufweist, die den gleichen Durchmesser haben, mit einem gemeinsamen Lumen, so dass der unbeschichtete Abschnitt mit dem gleichen Druck dilatiert wird wie der beschichtete Abschnitt. In einer Modifikation dieser Ausführung hat der unbeschichtete Abschnitt einen kleineren Durchmesser als der beschichtete, oder er verjüngt sich zu seinem Ende hin.

Der beschichtete Ballonbereich ist geschützt mit einer rückziehbaren Schutzhülle. Diese kann sich selbstständig zurückziehen oder manuell zurückgezogen werden.

Mit der Erfindung sind gegenüber bekannten Ballonkathetern mit medikamentenbeschichtetem Ballon erhebliche klinische Vorteile erreichbar; Insbesondere tritt kein oder zumindest ein geringfügiger Partikelverlust auf dem Weg zur Stenose und bei der Dilatation auf, und die Wirkstoffverteilung am Zielort lässt sich vorhersagbarer und homogener gestalten. Dies bietet die Möglichkeit der Realisierung kleinerer Dosierungen, mit dem zusätzlichen Vorteil einer geringeren systemischen Wirkstoffabgabe. Die offensichtlichen klinischen Vorteile führen zu ebenso deutlich hervortretenden Verbesserungen der Produkteigenschaften und damit der Marktaussichten des Produktes.

In einer weiteren Ausführung der Erfindung ist die Lage des medikamentenbeschichteten Ballons oder von dessen medikamentenbeschichteten Bereich, insbesondere eines Endes desselben, auf dem Katheterkörper mit mindestens einem Röntgenmarker markiert. Insbesondere ist vorgesehen, dass die Enden des medikamentenbeschichteten Ballons und die Grenze zwischen dem medikamentenbeschichteten und dem oder jedem medikamentenfreien Bereich oder die Enden des medikamentenbeschichteten Ballons und die Enden des oder jedes medikamentenfreien Bereichs jeweils mit einem Röntgenmarker markiert sind. Während bei einer Ausführung, bei der das (im Gebrauchszustand) distale Ende des Ballons oder ein zusätzlicher distaler Ballon frei von einer Medikamentenbeschichtung ist, das Zurückziehen der Schutzhülle in üblicher Weise von distal nach proximal durch proximales Erfassen der Schutzhülle erfolgen kann, erfordert das Zurückziehen der Schutzhülle bei proximal angeordnetem medikamentenfreien Bereich oder Ballon ein anderes Vorgehen: Die Schutzhülle muss hier von proximal nach distal zurückgezogen werden. Hierzu sind bei dieser Ausführung im Katheterkörper Verschiebemittel zum Verschieben der Schutzhülle zum Freilegen des medikamentenbeschichteten Ballons oder medikamentenbeschichteten Bereiches zum distalen Ende hin vorgesehen, derart, dass vor einem Freilegen des medikamentenbeschichteten Ballons oder medikamentenbeschichteten Bereichs der proximal angeordnete medikamentenfreie Ballon oder Abschnitt freigelegt wird. Speziell umfassen die Verschiebemittel einen Einschubdraht oder ein Schubrohr, welches bis zum proximalen Ende des Katheterkörpers reicht und dort mit einer Betätigungseinrichtung versehen ist.

Gemäß der Erfindung ist es vorgesehen, dass das dem medikamentenfreien Ballon oder medikamentenfreien Bereich zugewandte Ende der Schutzhülle flexibel aufweitbar ausgeführt ist. Diese Spitze schützt die Beschichtung beim Öffnen des beschichteten Ballons, bis sie an der Gefässwand anliegt. Das flexible Material der Spitze hat nach der Intervention keinen Nachteil beim Entfernen des Katheters.

Vorteile und Zweckmäßigkeiten der Erfindung werden im Übrigen aus der nachfolgenden Beschreibung von Ausführungsbeispielen anhand der Figuren deutlich. Es zeigen:
Fig. 1A bis 1D schematische Darstellungen zur Erläuterung einer ersten Ausführungsform der Erfindung,
Fig. 2A bis 2C schematische Darstellungen zur Erläuterung einer zweiten Ausführungsform der Erfindung und
Fig. 3A bis 3C schematische Darstellungen zur Erläuterung weiterer Ausführungsformen.

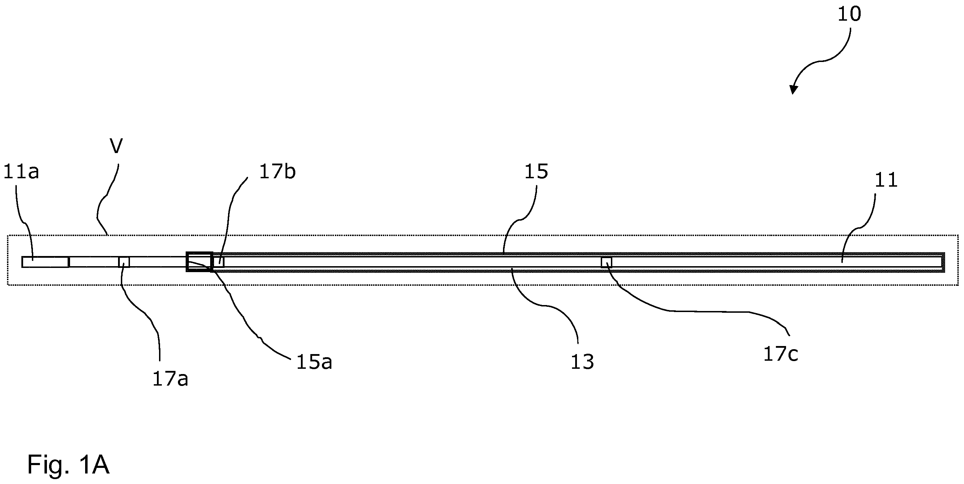

Fig. 1A bis 1D zeigen jeweils schematisch eine Phase des Einsatzes eines erfindungsgemäßen Ballonkatheters 10 in einem Gefäß V eines menschlichen (oder tierischen) Körpers.

Der Ballonkatheter 10 umfasst einen langgestreckten Katheterkörper 11 mit einem distalen Ende 11a und einem proximalen Ende, welches außerhalb des Körpers und somit außerhalb des Darstellungsbereiches der Zeichnung liegt. Auf dem Katheterkörper 11 ist ein Ballon 13 angebracht, der im Ausgangszustand (Fig. 1A) zusammengefaltet und einer verschieblichen Schutzhülle 15 angeordnet ist. Diese hat an ihrem distalen Ende eine flexibel aufweitbare, atraumatische Spitze 15a. Röntgenmarkierungsringe 17a, 17b und 17c sind zur Kennzeichnung bestimmter Abschnitte des Ballons (siehe weiter unten) vorgesehen.

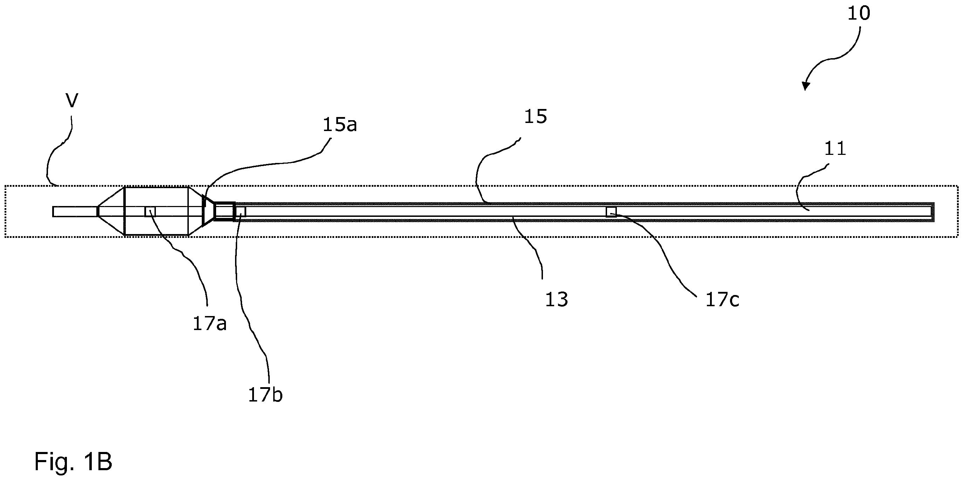

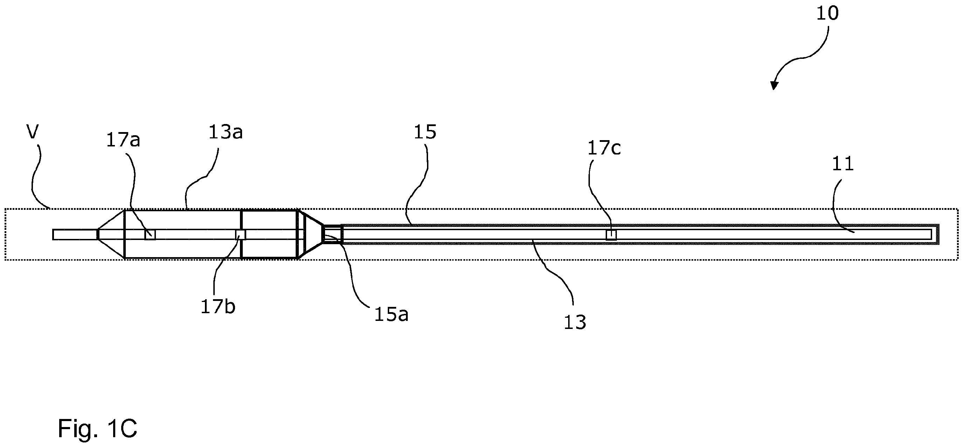

Während in Fig. 1A der Zustand des Ballonkatheters unmittelbar nach Einführung an den Einsatzort, also mit ursprünglicher Position der Schutzhülle 15, gezeigt ist, zeigen die Figuren 1B bis 1D verschiedene Phasen des Zurückziehens der Schutzhülle und damit einhergehenden Aufweitens des Ballons. In Fig. 1B ist die Schutzhülle 15 um einen relativ kleinen Betrag zurückgezogen und gibt einen kleinen distalen Bereich des Ballons 13 frei. Es ist hier auch zu erkennen, dass sich die Spitze 15a der Schutzhülle beim Zurückziehen und gleichzeitigen Aufweiten des Ballons selbst aufweitet. Im Zustand gemäß Fig. 1C ist der Ballon über einen größeren Teil seiner Länge expandiert, und die Schutzhülle ist entsprechend weiter zurückgezogen.

Hier ist auch dargestellt, dass ein distaler Abschnitt 13a des Ballons von einem proximalen Abschnitt 13b abgegrenzt ist, und zwar zur Verdeutlichung dessen, dass der distale Abschnitt 13a frei von einer Medikamentenbeschichtung ist. Mittels des Röntgenmarkierungsringes 17b ist die Grenze zwischen dem unbeschichteten distalen Ballonabschnitt 13a und dem beschichteten proximalen Abschnitt 13b auch für Röntgendarstellungsverfahren markiert. Die Spitze 15a ist hier stärker aufgeweitet als in dem in Fig. 1B gezeigten Zustand. In dieser Darstellung ist auch zu erkennen, dass der Röntgenmarkierungsring 17c das proximale Ende des beschichteten Ballonabschnitts 13b markiert. An diesen schließt sich weiter proximal noch ein kurzer unbeschichteter Ballonabschnitt 13c an, auf den der proximale Ballonkonus (nicht gesondert bezeichnet) folgt.

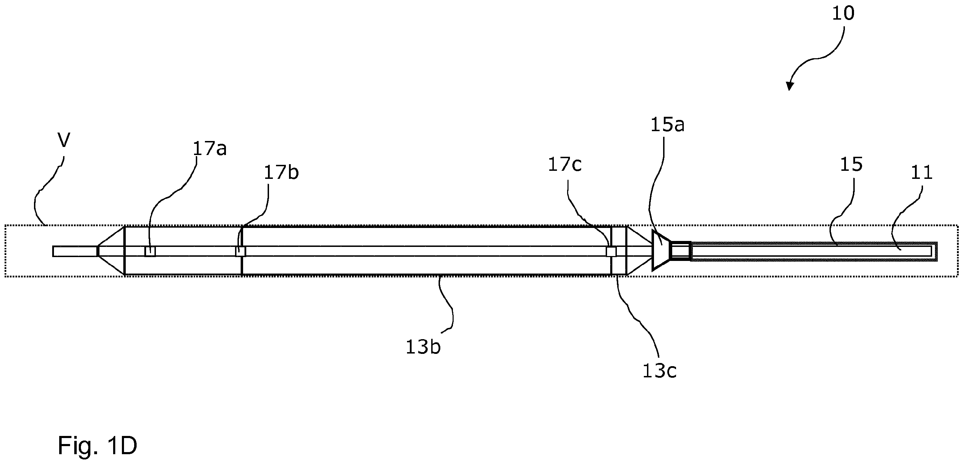

Fig. 1D zeigt den voll expandierten Zustand des Ballons 13 mit entsprechend weit zurückgezogener Schutzhülle 15. Die Spitze 15a der Schutzhülle ist hier noch aufgeweitet dargestellt, zieht sich aber in diesem Zustand des Ballonkatheters wieder zusammen, wodurch Gefäßverletzungen beim Zurückziehen des Katheters vermieden werden.

Es ist zu erkennen, dass bei dem in den Figuren 1A bis 1D dargestellten Vorgehen zunächst das Gefäß mit dem distalen unbeschichteten Ballonabschnitt 13a verschlossen wird, ehe der proximale Ballonabschnitt 13b expandiert und somit die Medikamentenbeschichtung in diesem Abschnitt freigelegt wird. Hierdurch wird ein Transport von Partikeln aus der Medikamentenbeschichtung verhindert. Stattdessen lagert sich das Medikament bei voll expandiertem Ballonabschnitt 13b in die Gefäßwandung ein, bevor der Ballon wieder deflatiert und der Ballonkatheter zurückgezogen wird.

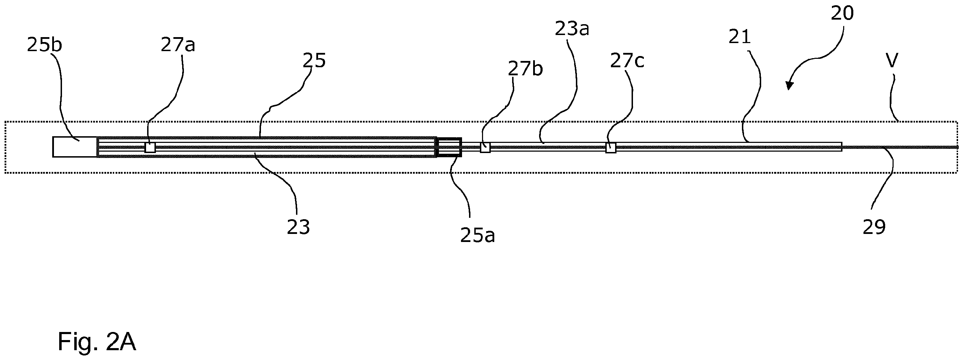

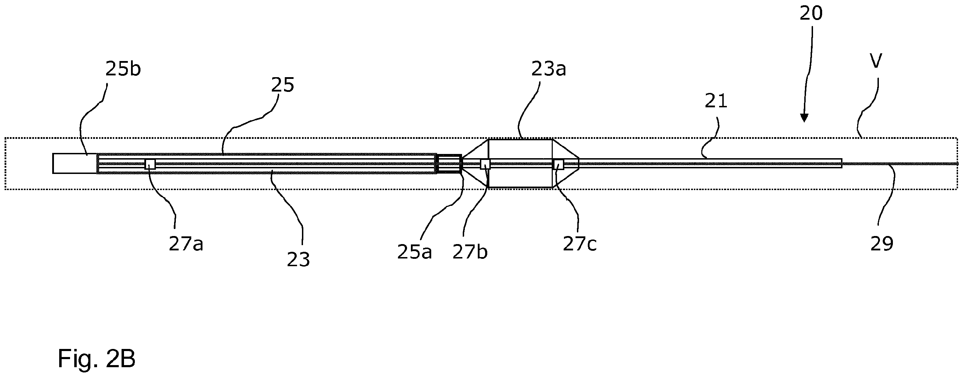

Fig. 2A bis 2C zeigen als weitere Ausführung der Erfindung einen Ballonkatheter 20, dessen Bestandteile in Anlehnung an die in Fig. 1A bis 1D gezeigte und oben beschriebene Ausführung mit Bezugsziffern versehen sind und hier nur insoweit nochmals beschrieben werden, als es die Erläuterung der abweichenden Merkmale dieser Ausführung erfordert. Ein wesentlicher Unterschied besteht darin, dass hier der proximale Endabschnitt 23a des Ballons 23 medikamentenfrei ist, und hieraus ergibt sich der weitere wesentliche Unterschied, dass die Schutzhülle 25 nicht von distal nach proximal, sondern von proximal nach distal verschieblich ausgeführt ist. Damit wird realisiert, dass zuerst der unbeschichtete proximale Ballonabschnitt 23a expandiert und in Kontakt mit der Wandung des Gefäßes V gebracht werden kann, bevor die Aufweitung des Ballons 23 auf dessen distalen, beschichteten Abschnitt 23b erfasst. Dieser Zwischenzustand ist in Fig. 2B dargestellt.

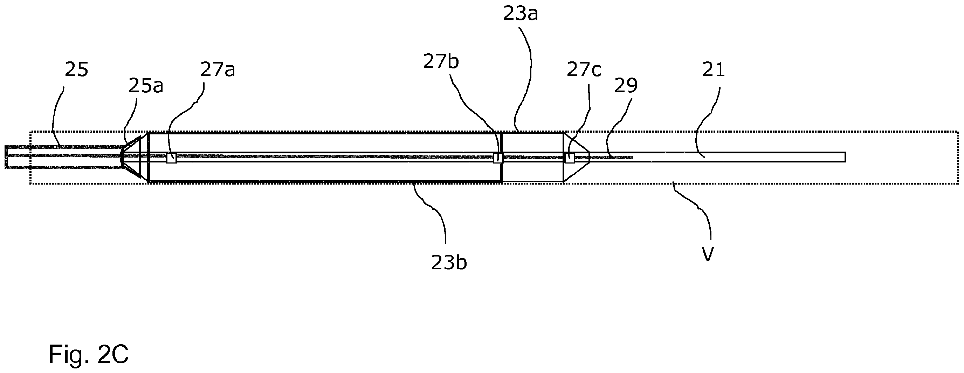

Fig. 2C zeigt den Endzustand der vollständigen Aufweitung der gesamten Länge des Ballons 23, einschließlich seines distalen beschichteten Abschnitts 23b. Die Röntgenmarkierungsringe 27a, 27b und 27c markieren hier wiederum wesentliche Abschnitte des Ballons, und zwar in dieser Reihenfolge das distale Ballonende, die Grenze zwischen beschichtetem und unbeschichtetem Abschnitt und das proximale Ballonende. Um die Schutzhülle von proximal nach distal verschieben zu können, ist im Katheterkörper 21 ein Schubrohr bzw. -draht 29 vorgesehen, der bis zum proximalen Ende des Ballonkatheters 20 reicht und von dort aus nach distal geschoben werden kann. Sein distales Ende ist am distalen Ende 25b der verschiebbaren Schutzhülle 25 befestigt, so dass er die Schutzhülle in distaler Richtung mitnimmt.

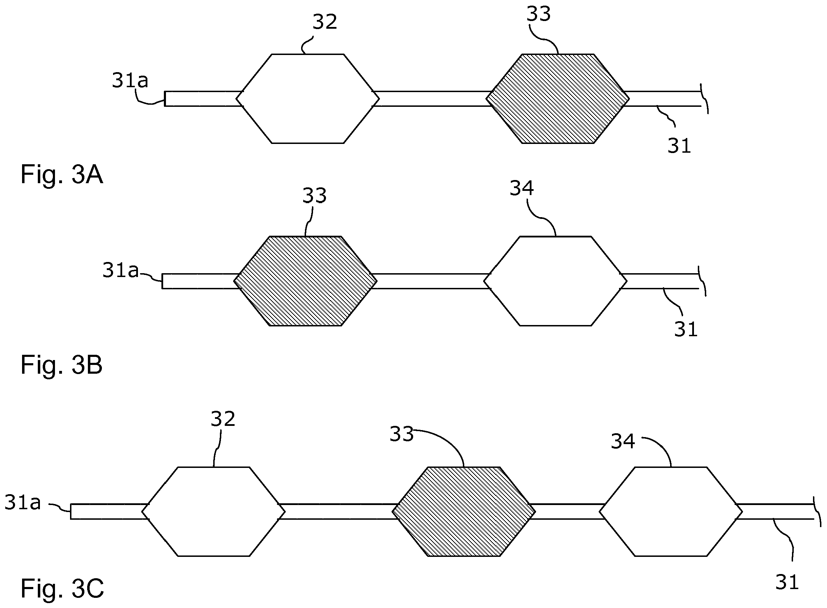

Fig. 3A zeigt skizzenartig als weitere Ausführung einen Ballonkatheter 30 mit zwei separaten Ballonen 32 und 33 auf einem Katheterkörper 31, von denen der proximal angeordnete Ballon 33 medikamentenbeschichtet, der distal angeordnete Ballon 32 hingegen frei von einer Medikamentenbeschichtung ist. Fig. 3B zeigt als weitere Ausführung einen Ballonkatheter 30', der ebenfalls zwei Ballone 33 und 34 aufweist, wobei hier der beschichtete Ballon 33 distal angeordnet ist. Fig. 3C schließlich zeigt einen weiteren Ballonkatheter 30", der drei Ballone 32, 33 und 34 umfasst, wobei sowohl distal als auch proximal vom beschichteten Ballon 33 jeweils ein unbeschichteter Ballon 32 bzw. 34 angeordnet ist.

Eine spezifische Ausgestaltung sieht vor, die Eigenschaften von expandiertem PTFE (e-PTFE) zu nutzen. Ein e-PTFE-Schlauch, der in seinem Aussendurchmesser dem schützenden Aussenschlauch entspricht, wird an diesem durch eine Klebeverbindung befestigt. e-PTFE lässt sich im Gegensatz zu anderen PTFE-Typen sehr leicht kleben, sofern Klebstoffe verwandt werden, welche niedrigviskos sind und die Fähigkeit besitzen unpolare Oberflächen zu benetzen. In diesem Falle erfolgt primär eine mechanische Verankerung der Mikrostruktur des e-PTFE mit dem penetrierenden Klebstoff. Es wird eine sehr zuverlässige Klebung/Verankerung des e-PTFE-Bauteiles mit dem Klebstoff erhalten.

In einem Beispiel kommen e-PTFE Schläuche der Firma Zeuss und der Cyanacrylat Klebstoff Loctite 4061 zum Einsatz. Der e-PTFE Schlauch wird mittels einer Telefonzange geweitet, um einen besseren Übergang von dem Schutzschlauch zu dem Tip-Segment zu realisieren. Alternativ kann statt von einem e-PTFE Schlauch natürlich auch von einer gerollten e-PTFE Folie ausgegangen werden. Die Verbindungsstelle wird mittels des Cyanacrylatklebers fixiert. Der Klebstoff Loctite 4061 benetzt den e-PTFE-Schlauch an den Kontaktstellen sehr gut und weist auch eine geeignete Viskosität auf, um die Porenstruktur des e-PTFE Schlauches zu infiltrieren.

Der überstehende Bereich wird in einer Isopropanol-Stearinsäure-Lösung getränkt, um eine über die Stearinsäure-Konzentration gesteuerte Menge der Fettsäure durch einen Tauchprozess auf das PTFE zu übertragen. Diese Lösung ist ab einer Temperatur oberhalb des Schmelzpunktes der verwandten Fettsäure Stearinsäure stabil. Anschliessend wird der getränkte Bereich in Heissluft ≥ 80°C getrocknet. Im vorliegenden Fall sollte ebenfalls eine Temperatur höher als der Schmelzpunkt der Fettsäure genutzt werden, da diese eine hohe Kristallisationsneigung aufweist und bei zu geringer Temperatur einen kristallinen, wenig mit dem e-PTFE-Substrat verbundenen Niederschlag erzeugt.

Bei diesem Prozessschritt wird das e-PTFE mittels der Stearinsäure infiltriert und wird über thermische Formprozesse formbar. Der erstellte Verbund kann nun mittels Temperaturen oberhalb des Schmelzpunktes der verwandten Fettsäure über Druck in eine temporäre Form gebracht werden.

Unter Druck ist dieser Verbund relativ stabil, unter Zug delaminieren die PTFE Bereiche, und der e-PTFE Schlauch wird wieder freigesetzt. Je nach Herstellung des e-PTFE kann dieser noch verformbar sein. Die primär genutzten Eigenschaften sind jedoch die Ausbildung eines Verbundes, der unter Druckbelastung stabil ist und unter Zugbeanspruchungen in den mittels der Fettsäure "verklebten" Bereichen unter Zugbelastung leicht delaminiert. Der delaminierte Verbund weist geringe Reibung, je nach Formulierung auch eine gewisse Weichheit, auf, was diesen konstruktiven Bereich des Katheters auch atraumatisch macht.

So sind Formulierungen möglich, die die Abformung des e-PTFE Verbundes zu einem atraumatischen geformten Tip oder Kathetersegmentes ermöglichen, welcher nach seiner plastischen Verformung keine bedenklich steifen und möglicherweise scharfkantige verformten Bereiche erzeugt. Solch ein Kathetersegment kann ohne Probleme durch eine enge Passage wie z.B. einen Introducer oder eine hemostatische Schleuse zurückgezogen werden, obwohl dieses Kathetersegment zuvor auf grössere Durchmesser verformt wurde.

Durch seine wiedererlangte Weichheit schützt dieser Verbund nach der Verformung auch vor Schädigungen, welche durch Gefäßkontakt erzeugt werden können.

Bei vorliegender Applikation soll der Verbund eine Katheterspitze formen, welche von innen aufgedrückt wird (durch einen dilatierenden Ballon). Hierbei delaminieren die verklebten Bereiche des Verbundes unter der entstehenden Zugbelastung. Wird eine noch weiter expandierbare e-PTFE Membrantype verwandt, kann dieser Tip noch erheblich geweitet werden (nach derzeitigen Versuchen ca. um den Faktor 4 des Durchmessers). Durch die Deformation delaminiert auch die Mikrostruktur des e-PTFE-Verbundes, und die Membran erhält annähernd ihre ursprüngliche Weichheit wieder.

Bei der Verformung durch einen innenliegenden Ballon kann primär durch die Länge des verwandten e-PTFE Schlauches die Verformung so bemessen werden, dass durch die verformte Spitze ein schützender Trichter entsteht, der vor dem Durchmesser welcher einen Kontakt zur Gefässwand erzeugen würde, endet.

Vorteilhafte Nebeneffekte werden wie folgt zusammengefasst:
- Das Kathetersegment kann plastisch verformt werden. Diese Verformung erzeugt ein weiches, flexibles und somit auch atraumatisches Gebilde. Die durch die Umformung erzeugte Flexibilität dieses Konstruktes macht Deformation z.B. für den Rückzug und das weitere Handling des Katheters unproblematisch.
- Das e-PTFE und wie auch der Stearinsäure-beschichtete Verbund zeichnet sich durch eine reibungsarme Oberfläche aus.
- Unter dem Mikroskop ist eine minimale Partikelbildungen bei der Verwendung reiner Stearinsäure nachweisbar. Die Fixierung der Fettsäure in der PTFE Struktur und an der Oberfläche kann als sehr effektiv betrachtet werden. Durch Reduktion der Kristallinität dieses Bindesystemes - bei Stearinsäure z.B. durch Zumischung von Ölsäure - können Anforderungen an Partikelbildung und auch Reduktion der Verbundfestigkeit in einfacher Form optimiert werden.

## Patentansprüche

1. Ballonkatheter (10; 20) mit einem medikamentenbeschichteten Ballon (13; 23), der in einem Ausgangszustand mit einer verschieblichen Schutzhülle (15; 25) bedeckt ist, auf einem langgestreckten Katheterkörper (11; 21), der ein distales und ein proximales Ende (13a, 13b; 23a, 23b) hat, wobei der distale und/oder proximale Bereich des medikamentenbeschichteten Ballons frei von einer Medikamentenbeschichtung ist und wobei das dem medikamentenfreien Bereich (13a; 23a) zugewandte Ende (15a; 25a) der Schutzhülle (15; 25) flexibel aufweitbar ausgeführt ist.

2. Ballonkatheter nach Anspruch 1, wobei der von der Medikamentenbeschichtung freie distale Bereich des Ballons einen kleineren Durchmesser als der verbleibende Bereich hat und/oder sich zu seinem distalen Ende hin verjüngt.

3. Ballonkatheter (10; 20) nach einem der vorangehenden Ansprüche, wobei die Lage des medikamentenbeschichteten Ballons oder von dessen medikamentenbeschichteten Bereich (13b; 23b), insbesondere eines Endes desselben, auf dem Katheterkörper (11; 21) mit mindestens einem Röntgenmarker (17a, 17b, 17c; 27a, 27b, 27c) markiert ist.

4. Ballonkatheter (10; 20) nach Anspruch 3, wobei die Enden des medikamentenbeschichteten Ballons und die Grenze zwischen dem medikamentenbeschichteten (13b; 23b) und dem oder jedem medikamentenfreien Bereich (13a; 23a) oder die Enden des medikamentenbeschichteten Ballons und die Enden des oder jedes medikamentenfreien Bereichs jeweils mit einem Röntgenmarker (17a, 17b, 17c; 27a, 27b, 27c) markiert sind.

5. Ballonkatheter (20) nach einem der vorangehenden Ansprüche, wobei im Katheterkörper Verschiebemittel (29) zum Verschieben der Schutzhülle (25) zum Freilegen des medikamentenbeschichteten Bereiches (23b) zum distalen Ende hin vorgesehen sind, derart, dass vor einem Freilegen des medikamentenbeschichteten Bereichs der proximal angeordnete medikamentenfreie Bereich (23a) freigelegt wird.

6. Ballonkatheter (20) nach Anspruch 5, wobei die Verschiebemittel einen Schubdraht oder ein Schubrohr (29) umfassen, welcher/welches bis zum proximalen Ende des Katheterkörpers (21) reicht und dort mit einer Betätigungseinrichtung versehen ist.

## Claims

1. A balloon catheter (10; 20) comprising a drug-coated balloon (13; 23), which in a starting state is covered by a displaceable protective sleeve (15; 25), on an elongate catheter body (11; 21), which has a distal end and a proximal end (13a, 13b; 23a, 23b), wherein the distal and/or proximal region of the drug-coated balloon is free from a drug coating, and wherein the end (15a; 25a) of the protective sleeve (15; 25) facing the drug-free region (13a; 23a) is expandable in a flexible manner.

2. The balloon catheter according to claim 1, wherein the distal region of the balloon, free from the drug coating, has a smaller diameter than the remaining region and/or tapers toward its distal end.

3. The balloon catheter (10; 20) according to one of the preceding claims, wherein the position of the drug-coated balloon or of the drug-coated region (13b; 23b) thereof, in particular an end thereof, is marked on the catheter body (11; 21) by at least one X-ray marker (17a, 17b, 17c; 27a, 27b, 27c).

4. The balloon catheter (10; 20) according to claim 3, wherein the ends of the drug-coated balloon and the borders between the drug-coated (13b; 23b) region and the, or each, drug-free region (13a; 23a) or the ends of the drug-coated balloon and the ends of the, or each, drug-free region are each marked by an X-ray marker (17a, 17b, 17c; 27a, 27b, 27c).

5. The balloon catheter (20) according to one of the preceding claims, wherein displacement means (29) for displacing the protective sleeve (25) toward the distal end to expose the drug-coated region (23b) are provided in the catheter body, in such a way that the proximally arranged drug-free region (23a) is exposed before the drug-coated region is exposed.

6. The balloon catheter (20) according to claim 5, wherein the displacement means comprise a push wire or a push rod (29), which reaches the proximal end of the catheter body (21), where it is provided with an actuation device.

## Revendications

1. Cathéter à ballonnet (10; 20) doté d'un ballonnet (13; 23) revêtu de médicaments qui est recouvert avec une enveloppe de protection (15 ; 25) pouvant se déplacer vers un état de départ, sur un corps de cathéter (11 ; 21) étiré en longueur, qui a une extrémité distale et une extrémité proximale (13a, 13b; 23a, 23b), où la région distale, et/ou la région proximale, du ballonnet revêtu de médicaments est exempte de revêtement de médicaments, et où l'extrémité (15a; 25a) de l'enveloppe de protection (15 ; 25) orientée vers la région exempte de médicaments (13a ; 23a) est conçue en pouvant s'élargir de manière flexible.

2. Cathéter à ballonnet selon la revendication 1, dans lequel la région distale du ballonnet exempte du revêtement de médicaments a un diamètre plus petit que la région restante et/ou s'amenuise vers son extrémité distale.

3. Cathéter à ballonnet (10; 20) selon l'une des revendications précédentes, dans lequel la position du ballonnet revêtu de médicaments ou de sa région (13b ; 23b) revêtue de médicaments, en particulier une extrémité de celui-ci, est marquée sur le corps de cathéter (11 ; 21) avec au moins un marqueur de rayons X (17a, 17b, 17c ; 27a, 27b, 27c).

4. Cathéter à ballonnet (10 ; 20) selon la revendication 3, dans lequel les extrémités du ballonnet revêtu de médicaments et la limite entre la région revêtue de médicaments (13b ; 23b) et la région ou chaque région exempte de médicaments (13a ; 23a), ou les extrémités du ballonnet revêtu de médicaments et les extrémités de la région ou de chaque région exempte de médicaments, sont respectivement marquées avec un marqueur de rayons X (17a, 17b, 17c ; 27a, 27b, 27c).

5. Cathéter à ballonnet (20) selon l'une des revendications précédentes, dans lequel des systèmes de déplacement (29) pour le déplacement de l'enveloppe de protection (25) pour la mise à nu de la région (23b) revêtue de médicaments vers l'extrémité distale sont prévus dans le corps de cathéter, de telle manière, qu'avant la mise à nu de la région revêtue de médicaments, la région exempte de médicaments (23a) disposée de manière proximale est mise à nu.

6. Cathéter à ballonnet (20) selon la revendication 5, dans lequel les systèmes de déplacement comprennent un fil de poussée ou un tube de poussée (29), lequel atteint l'extrémité proximale du corps de cathéter (21) et est muni d'un dispositif d'actionnement à cet endroit.
